# EUROPEAN PATENT APPLICATION

(11) **EP 2 612 879 A1**
(43) Date of publication of application: **10.07.2013**
(21) Application number: 12812064.9
(22) Date of filing: 06.07.2012
(51) Int. Cl.: C08G 73/10, A61K 6/087, B32B 15/088, B32B 18/00, B32B 27/34, C08L 79/08, C09J 179/08, H01L 23/29, H01L 23/31, H01L 31/042, H01M 2/16, H01M 4/62, H05K 1/03

(54) **POLYIMIDE RESIN COMPOSITION AND LAMINATE INCLUDING SAME**

(30) Priority: 08.07.2011 JP 2011151919; 20.01.2012 JP 2012010167; 26.04.2012 JP 2012101504
(71) Applicant: Mitsui Chemicals, Inc., Tokyo 105-7117 (JP)
(72) Inventor: IIDA, Kenji, Chiba 299-0265 (JP); TOMITA, Yusuke, Chiba 299-0265 (JP); IMAGAWA, Kiyomi, Chiba 299-0265 (JP); KIBA, Shigeo, Chiba 299-0265 (JP)
(74) Representative: HOFFMANN EITLE
(86) International application number: PCT/JP2012/004406
(87) International publication number: WO 2013/008437

(57) **Abstract**

To provide a resin composition that contains a solvent-soluble polyimide and can provide a film exhibiting high viscoelasticity and flexibility at high temperatures. To attain this, a polyimide resin composition is provided that includes a polyimide having a polycondensation unit of a tetracarboxylic acid dianhydride and a diamine, wherein the tetracarboxylic acid dianhydride includes an (α1) tetracarboxylic acid dianhydride represented by general formula (1), or the diamine includes an (β1) aromatic diamine represented by general formula (2), the diamine includes an (β2) aliphatic diamine represented by general formula (3) or (4), a total amout of the (α1) tetracarboxylic acid dianhydride and the (β1) aromatic diamine is 5 to 49 mol% with respect to a total amount of the tetracarboxylic acid dianhydride and the diamine, and an amine equivalent of the polyimide is 4,000 to 20,000.

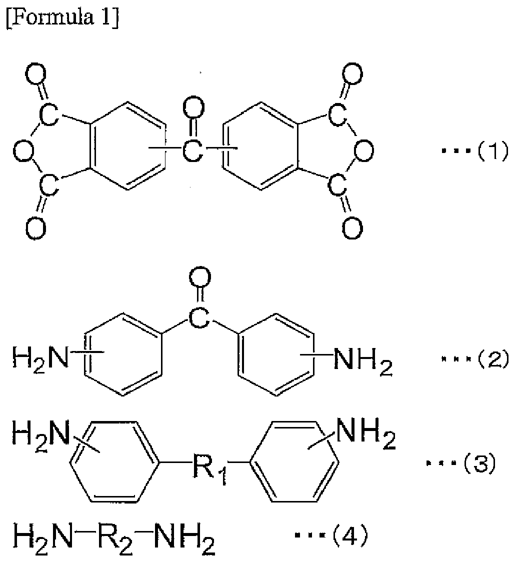

## Description

### Technical Field

The present invention relates to polyimide resin compositions and laminates containing the polyimide resin composition.

### Background Art

Conventionally, epoxy resins have been used for adhesives for electronic circuit boards, semiconductor devices, and other devices. However, epoxy resins lack sufficient heat resistance and/or flexibility, and require a long thermal curing reaction time.

On the other hand, thermoplastic polyimide resins are not only known to exhibit high heat resistance and flexibility, but also to require a relatively short thermal curing reaction time. However, because thermoplastic polyimide resins are normally obtained by imidization of coated films of polyamic acid varnish at temperatures as high as 300°C or above, applicable processes and/or members for the thermoplastic polyimide resins have been limited.

Methods of obtaining a thermoplastic polyimide resin without the high-temperature imidization process include drying a coated film of varnish in which a polyimide is dissolved in solvent (i.e., a varnish of solvent-soluble polyimide) (see, e.g., Patent Literatures 1 and 2). Patent Literature 1 discloses a solvent-soluble polyimide obtained by reacting an acid dianhydride component including benzophenone tetracarboxylic acid dianhydride with a diamine component including a specific siloxane compound. Patent Literature 2 discloses a solvent-soluble polyimide obtained by reacting an acid dianhydride component including benzophenone tetracarboxylic acid dianhydride with a diamine component including a compound having a specific sulfonic acid backbone.

### Citation List

### Patent Literature

PTL 1: Japanese Patent Application Laid-Open No. 09-255780
PTL 2: Japanese Patent Application Laid-Open No. 2001-310336

### Summary of Invention

### Technical Problem

However, a film obtained from the polyimide disclosed by Patent Literature 1 lacked sufficient flexibility. Moreover, although a film obtained from the polyimide disclosed by Patent Literature 2 exhibited relatively high heat resistance due to the presence of a diamine having a sulfonic acid backbone, flexibility was low Accordingly, resin compositions containing these polyimides were not suited for applications in which flexibility was required.

In order to obtain a polyimide that is solvent-soluble and that provides high film flexibility, prevention of packing of imide rings in the polyimide by the introduction of a long chain alkyleneoxy group or other group between every two of the imide rings is typically effective. However, films obtained from polyimides having a long chain alkyleneoxy group have the drawbacks of low viscoelasticity at high temperatures and low heat resistance.

The present invention has been made in view of the foregoing problems pertinent in the art, and an object of the present invention is to provide a resin composition containing a polyimide that is solvent-soluble and that provides a film that exhibits high viscoelasticity and flexibility at high temperatures.

### Solution to Problem

As previously described, although a polyimide having an alkyleneoxy chain is solvent-soluble and a film obtained from the polyimide exhibits high flexibility, heat resistance is insufficient. On the other hand, by introducing a benzophenone backbone into the polyimide and introducing an amino group as the molecular terminal group, the carbonyl group in the benzophenone backbone and the amino group as the molecular terminal group can be hydrogen bonded, whereby heat resistance can be increased. As a result, a polyimide film can be obtained that has high heat resistance and flexibility.

More specifically, a first aspect of the present invention relates to the polyimide resin compositions given below.
[1] A polyimide resin composition including:
   a polyimide including a polycondensation unit of a tetracarboxylic acid dianhydride and a diamine, wherein
   the tetracarboxylic acid dianhydride constituting the polyimide includes an (α1) aromatic tetracarboxylic acid dianhydride having a benzophenone backbone represented by the following general formula (1), or the diamine constituting the polyimide includes an (β1) aromatic diamine having a benzophenone backbone represented by the following general formula (2),
   the diamine constituting the polyimide includes an (β2) aliphatic diamine represented by the following general formula (3) or (4),
   a total amount of the (α1) aromatic tetracarboxylic acid dianhydride having the benzophenone backbone represented by the general formula (1) and the (β1) aromatic diamine having the benzophenone backbone represented by the general formula (2) is 5 to 49 mol% with respect to a total amount of the tetracarboxylic acid dianhydride and the diamine constituting the polyimide, and
   an amine equivalent of the polyimide is 4,000 to 20,000, 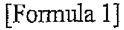 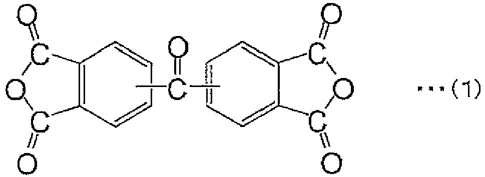 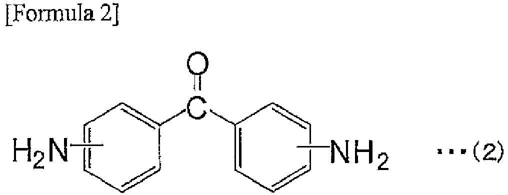 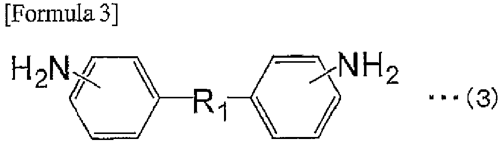 wherein in the formula (3), R₁ is an aliphatic chain including a main chain including at least one of C, N and O, a total number of atoms constituting the main chain is 7 to 500; and the aliphatic chain may also include a side chain including at least one C, N, H or O atom, and a total number of atoms constituting the side chain is 10 or less,
      [Formula 4]

      H₂N-R₂-NH₂ (4)

      wherein in the formula (4), R₂ is an aliphatic chain including a main chain including at least one of C, N and O, a total number of atoms constituting the main chain is 5 to 500; and the aliphatic chain may also include a side chain including at least one C, N, H or O atom, and a total number of atoms constituting the side chain is 10 or less.

[2] The polyimide resin composition according to [1], wherein a total number of moles of the tetracarboxylic acid dianhydride constituting the polyimide is 0.95 to 0.999 with respect to a total number of moles of the diamine constituting the polyimide.
[3] The polyimide resin composition according to [1] or [2], further including a solvent, wherein the polyimide is dissolved into the solvent.
[4] The polyimide resin composition according to any one of [1] to [3], wherein R₁ in the general formula (3) and R₂ in the general formula (4) are each an aliphatic chain having a main chain including an alkyleneoxy group or a polyalkyleneoxy group, and
   the number of carbon atoms of an alkylene moiety of the alkyleneoxy group and the number of carbon atoms of an alkylene moiety of an alkyleneoxy unit constituting the polyalkyleneoxy group are 1 to 10 each.
[5] The polyimide resin composition according to any one of [1] to [4], wherein the (β2) aliphatic diamine represented by the general formula (3) is a compound represented by the following general formula (3-1), and the (β2) aliphatic diamine represented by the general formula (4) is a compound represented by the following general formula (4-1) 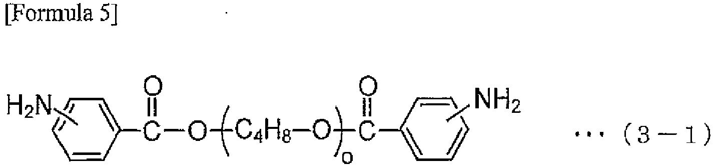 wherein in the formula (3-1), o represents an integer from 1 to 50, 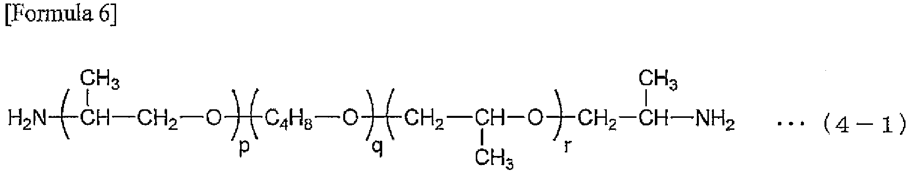 wherein, in the formula (4-1), p, q and r each independently represent an integer from 0 to 10, with the proviso that p+q+r is at least 1.

[6] The polyimide resin composition according to any one of [1] to [5], wherein the (β2) aliphatic diamine represented by the general formula (3) or (4) is present in an amount of 10 mol% to 45 mol% with respect to a total amount of the diamine constituting the polyimide.
[7] The polyimide resin composition according to any one of [1] to [6], wherein the (α1) aromatic tetracarboxylic acid dianhydride having a benzophenone backbone represented by the general formula (1) is at least one compound selected from the group consisting of 3,3',4,4'-benzophenone tetracarboxylic acid dianhydride, and 2,3,3',4'-benzophenone tetracarboxylic acid dianhydride, and
   the (β1) aromatic diamine having a benzophenone backbone represented by the general formula (2) is at least one compound selected from the group consisting of 3,3'-diaminobenzophenone, 3,4-diaminobenzophenone, and 4,4'-diaminobenzophenone.
[8] The polyimide resin composition according to any one of [1] to [7], wherein the polyimide has a glass transition temperature of 120°C to less than 260°C.
[9] The polyimide resin composition according to any one of [1] to [8], further including an inorganic filler.

[10] The polyimide resin composition according to [9], wherein the inorganic filler is a heat-dissipating filler, and
   a volume of the heat-dissipating filler is 20 to 60vol% with respect to a total volume of the polyimide resin composition.
[11] The polyimide resin composition according to [9] or [10], wherein the inorganic filler is an electroconductive filler and/or a magnetic filler, and
   a total volume of the electroconductive filler and magnetic filler is 20 to 90vol% with respect to the total volume of the polyimide resin composition.
[12] The polyimide resin composition according to [1], further including at least one compound selected from the group consisting of an epoxy compound, an acrylate compound, an isocyanate compound, a maleimide compound, and a nadimide compound.

[13] A laminate including:
   a substrate and;
   a resin layer disposed on the substrate, the resin layer being formed of the polyimide resin composition according to any one of [1] to [12].
[14] The laminate according to [13], wherein the substrate is made of metal, ceramic or resin.
[15] An electronic circuit board member including the laminate according to [13] or [14].
[16] A semiconductor device including the laminate according to [13] or [14].

[17] An electrode for a lithium-ion battery, including:
   a metal foil; and
   a layer disposed on the metal foil, the layer including an active substance and the polyimide resin composition according to any one of [1] to [12].
[18] A separator for a lithium-ion battery including the polyimide resin composition according to any one of [1] to [12].
[19] A heat-dissipating substrate including the polyimide resin composition according to [10].
[20] An electromagnetic shielding substrate including the polyimide resin composition according to [11].
[21] An adhesive for a surge part including the polyimide resin composition according to any one of [1] to [12].
[22] A sealant for a surge part including the polyimide resin composition according to any one of [1] to [12].
[23] An adhesive for a semiconductor manufacturing device including the polyimide resin composition according to any one of [1] to [12].
[24] A dental material including the polyimide resin composition according to any one of [1] to [12].

[25] A polyimide resin composition including a polyimide which is soluble in polar solvent, wherein
   a polyimide film having a thickness of 50 µm which is formed of the polyimide resin composition satisfies the following conditions a) and b):
      a) a storage modulus of elasticity E' measured at 180°C and a frequency of 1 Hz is 1.0×10⁵ Pa or more, and
      b) an elongation rate at the time of tensile fracture at 23°C at a speed of 50 mm/min is 50% or more.

### Advantageous Effects of Invention

A polyimide resin composition of the present invention is superior in solvent-solubility, and exhibits high viscoelasticity and high flexibility at high temperatures. Accordingly, the polyimide resin composition is suitable as an adhesive and the like in various fields in which high heat resistance and flexibility are required, including electronic circuit board members, semiconductor devices, lithium-ion battery members, and solar cell members).

### Brief Description of Drawings

FIG. 1 is a schematic view showing an example of ball grid array (BGA) packaging: and
FIG. 2 is a schematic view showing an example of chip-on-film (COP) packaging.

### Description of Embodiments

### 1. Polyimide resin composition

A polyimide resin composition of the present invention includes a specific polyimide, and may further include optional component(s) such as inorganic fillers when needed.

The polyimide contained in the polyimide resin composition includes a polycondensation unit of a tetracarboxylic acid dianhydride and a diamine, one feature of the polyimide is that 1) the tetracarboxylic acid dianhydride includes an (α1) tetracarboxylic acid dianhydride having a benzophenone backbone; the diamine includes a (β1) diamine having a benzophenone backbone; or the tetracarboxylic acid dianhydride, includes the (α1) tetracarboxylic acid dianhydride having a benzophenone backbone, and the diamine includes the (β1) diamine having a benzophenone backbone; and 2) the diamine includes an (β2) aliphatic diamine including an alkyleneoxy group.

The tetracarboxylic acid dianhydride constituting the polyimide may include the (α1) tetracarboxylic acid dianhydride having a benzophenone backbone. The (α1) tetracarboxylic acid dianhydride having a benzophenone backbone is preferably an aromatic tetracarboxylic acid dianhydride having a benzophenone backbone that is represented by general formula (1).

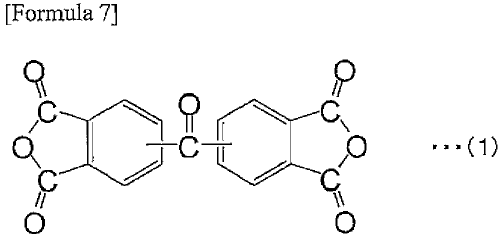

Examples of the aromatic tetracarboxylic acid dianhydride having a benzophenone backbone represented by the general formula (1) may include 3,3,4,4'-benzophenone tetracarboxylic acid dianhydride, 2,3,3',4'- benzophenone tetracarboxylic acid dianhydride, and 2,2',3,3'- benzophenone tetracarboxylic acid dianhydride. The above examples of the aromatic tetracarboxylic acid dianhydride having a benzophenone backbone represented by general formula (1) may be used either alone or in combination.

The tetracarboxylic acid dianhydride constituting the polyimide may further include a second (α2) tetracarboxylic acid dianhydride other than the aromatic tetracarboxylic acid dianhydride having a benzophenone backbone. Although the second (α2) tetracarboxylic acid dianhydride is not particularly limited, an aromatic tetracarboxylic acid dianhydride is preferably employed from the perspective of heat resistance, and an aliphatic tetracarboxylic acid dianhydride is preferably employed from the perspective of flexibility.

Examples of the aromatic tetracarboxylic acid dianhydride that may be employed as the second (α2) tetracarboxylic acid dianhydride may include pyromellitic dianhydride, 3,3',4,4-'biphenyltetracarboxylic acid dianhydride, 1,1',2,2-biphenyltetracarboxylic acid dianhydride, 2,3,2',3'-biphenyltetracarboxylic acid dianhydride, 1,2,2',3-biphenyltetracarboxylic acid dianhydride, bis(3,4-dicarboxyphenyl)ether dianhydride, bis(3,4-dicarboxyphenyl)sulfide dianhydride, bis(3,4-dicarboxyphenyl)sulfone dianhydride, bis(3,4-dicarboxyphenyl)methane dianhydride, 2,2-bis(3,4-dicarboxyphenyl)propane dianhydride, 2,2-bis(3,4-dicarboxyphenyl)-1,1,1,3,3,3-hexafluoropropane dianhydride, 1,3-bis(3,4-dicarboxyphenoxy)benzene dianhydride, 1,4-bis(3,4-dicarboxyphenoxy)benzene dianhydride, 4,4'-bis(3,4-dicarboxyphenoxy)biphenyl dianhydride, 2,2-bis[(3,4-dicarboxyphenoxy)phenyl]propane dianhydride, 2,3,6,7-naphthalene tetracarboxylic acid dianhydride, 1,4,5,8-naphthalene tetracarboxylic acid dianhydride, 2,2',3,3'-biphenyltetracarboxylic acid dianhydride, 2,2-bis(2,3-dicarboxyphenoxy)propane dianhydride, 2,2-bis(2,3-dicarboxyphenoxy)-1,1,1,3,3,3-hexafluoropropane dianhydride, bis(2,3-dicarboxyphenoxy)ether dianhydride, bis(2,3-dicarboxyphenoxy)sulfide dianhydride, bis(2,3-dicarboxyphenoxy)sulfone dianhydride, 1,3-bis(2,3-dicarboxyphenoxy)benzene dianhydride, 1,4-bis(2,3-dicarboxyphenoxy)benzene dianhydride, 1,2,5,6-naphthalene tetracarboxylic acid dianhydride, 4,4'-isopthaloyl diphthalic anhydride diazodiphenylmethane-3,3',4,4'-tetracarboxylic acid dianhydride, diazodiphenylmethane-2,2',3,3'-tetracarboxylic acid dianhydride, 2,3,6,7-thioxanthone tetracarboxylic acid dianhydride, 2,3,6,7-anthraquinone tetracarboxylic acid dianhydride, 2,3,6,7-xanthone tetracarboxylic acid dianhydride, and ethylene tetracarboxylic acid dianhydride.

Examples of the aliphatic tetracarboxylic acid dianhydride that may be employed as the second (α2) tetracarboxylic acid dianhydride may include cyclobutane tetracarboxylic acid dianhydride, 1,2,3,4-cyclopentane tetracarboxylic acid dianhydride, 1,2,4,5-cyclohexane tetracarboxylic acid dianhydride, bicyclo[2.2.1]heptane-2,3,5,6-tetracarboxylic acid dianhydride, bicyclo[2.2.2]oct-7-ene-2,3,5,6-tetracarboxylic acid dianhydride, bicyclo[2.2.2]octane-2,3,5,6-tetracarboxylic acid dianhydride, 2,3,5-tricarboxycyclopentyl acetic acid dianhydride, bicyclo[2.2.1]heptane-2,3,5-tricarboxylic acid-6-acetic acid dianhydride, 1-methyl-3-ethylcyclohexa-1-ene-3-(1,2),5,6-tetracarboxylic acid dianhydride, 4-(2,5-dioxotetrahydrofuran-3-yl)-tetralin-1,2-dicarboxylic acid dianhydride, and 3,3',4,4'-dicyclohexyltetracarboxylic acid dianhydride.

When the tetracarboxylic acid dianhydride constituting the polyimide includes an aromatic ring such as a benzene ring, some or all of the hydrogen atoms on the aromatic ring may be replaced by a group selected from fluoro group, methyl group, methoxy group, fluoromethyl group, trifluoromethoxy group and the like. Moreover, when the tetracarboxylic acid dianhydride includes an aromatic ring such as a benzene ring, the tetracarboxylic acid dianhydride may have a group that serves as a crosslinking point, which is selected from ethynyl group, benzocyclobutane-4'-yl group, vinyl group, allyl group, cyano group, isocyanate group, nitrilo group, isopropenyl group and the like, depending on the intended purpose. These groups may be used either alone or in combination.

In order to attain high heat resistance without significantly compromising flexibility, the second (α2) tetracarboxylic acid dianhydride is preferably an aromatic tetracarboxylic acid dianhydride, with 3,3',4,4'-biphenyltetracarboxylic acid dianhydride, 1,2,1',2'-biphenyltetracarboxylic acid dianhydride, 2,3,2',3'-biphenyltetracarboxylic acid dianhydride or 1,2,2',3-biphenyltetracarboxylic acid dianhydride being even more preferable.

The diamine constituting the polyimide may include an (β1) aromatic diamine having a benzophenone backbone. The (β1) aromatic diamine having a benzophenone backbone is preferably an aromatic diamine having a benzophenone backbone represented by general formula (2).

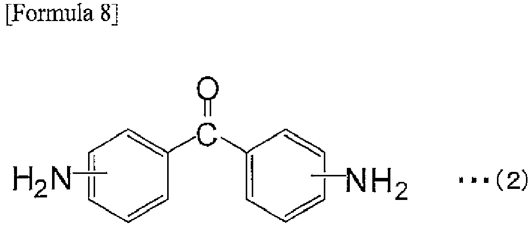

Examples of the aromatic diamine having a benzophenone backbone represented by the general formula (2) may include 3,3'-diaminobenzophenone, 3,4-diaminobenzophenone, and 4,4'-diaminobenzophenone. These aromatic diamines may be used either alone or in combination.

The diamine constituting the polyimide includes an (β2) aliphatic diamine. The (β2) aliphatic diamine is preferably an aliphatic diamine represented by general formula (3) or general formula (4). The polyimide resin composition may include either one or both of the aliphatic diamines respectively represented by the following general formulas (3) and (4). 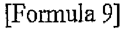 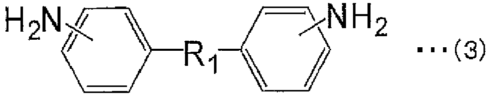 [Formula 10]

H₂N-R₂-NH₂ (4)

R₁ in the formula (3) and R₂ in the formula (4) represent an aliphatic chain having a main chain including at least one of C, N and O, and preferably an aliphatic chain having a main chain including at least one C atom. The total number of atoms constituting the main chain is preferably 5-500, more preferably 10-500, even more preferably 21-300, and most preferably 50-300. The main chain of R₁ in the general formula (3) refers to, in the aliphatic chain that links the two terminal phenyl groups, a moiety that consists of atom(s) other than those constituting a side chain of the aliphatic chain. The main chain of R₂ in the general formula (4) refers to, in the aliphatic chain that links the two terminal amino groups, a moiety that consists of atom(s) other than those constituting a side chain of the aliphatic chain.

Examples of the main chain that constitutes the aliphatic chain and that consists of at least one of C, N and O may include main chains having a structure derived from a polyalkylenepolyamine such as diethylenetriamine, triethylenetetramine or tetraethylenepentamine; main chains including an alkylene group; main chains having a polyalkyleneglycol structure; main chains having an alkylether structure; main chains having a polyalkylenecarbonate structure; and main chains including an alkyleneoxy group or a polyalkyleneoxy group, with main chains including an alkyleneoxy group or a polyalkyleneoxy group being preferable.

A polyalkyleneoxy group refers to a bivalent linking group which includes a repeat unit of alkyleneoxy; exemplary polyalkyleneoxy groups are "-(CH₂CH₂O)ₙ-" having ethyleneoxy unit as a repeat unit and "-(CH₂-CH(CH₃)O)ₘ-" having propyleneoxy unit as a repeat unit (where n and m represent a number of repeats). The number of repeats of the alkyleneoxy unit in the polyalkyleneoxy group is preferably 2-50, more preferably 2-20, and even more preferably 2-15. The polyalkyleneoxy group may include different alkyleneoxy units.

The number of carbon atoms in an alkylene moiety of an alkyleneoxy group and the number of carbon atoms in an alkylene moiety of an alkyleneoxy unit constituting a polyalkyleneoxy group are preferably 1-10 each, and more preferably 2-10 each. Examples of the alkylene group constituting the alkyleneoxy group may include methylene group, ethylene group, propylene group, and butylene group. The presence of butylene group as the alkylene group constituting the alkyleneoxy group or polyalkyleneoxy group advantageously allows a polyimide film obtained from the polyimide resin composition of the present invention to exhibit superior fracture strength.

There are no particular limitations to the group for linking the alkyleneoxy group or the polyalkyleneoxy group to the terminal amino group in the main chain of R₁ or R₂; it may be an alkylene group, an arylene group, an alkylene carbonyloxy group, or an arylene carbonyloxy group, with an alkylene group being preferable from the perspective of enhancing the reactivity of the terminal amino groups.

The aliphatic chains represented by R₁ and R₂ may further include a side chain including at least one of C, N, H and O. The side chain in the R₁ and R₂ is a monovalent group linked to an atom constituting the main chain. The total number of atoms constituting each side chain is preferably no more than 10. Examples of the side chain may include an alkyl group such as methyl group, as well as hydrogen atom.

Consequently, because the (β2) aliphatic diamine represented by the general formula (3) or (4) includes a long aliphatic chain, the resultant polyimide exhibits superior flexibility.

The aliphatic diamine represented by the general formula (3) is preferably a compound represented by general formula (3-1). The aliphatic diamine represented by the general formula (4) is preferably a compound represented by general formula (4-1).

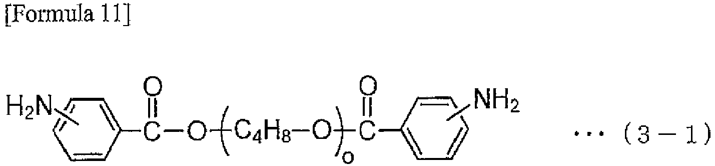

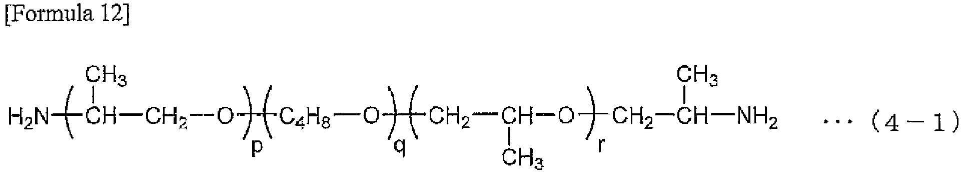

In the formula (3-1), o represents an integer of 1-50, and preferably an integer of 10-20. In the formula (4-1), p, q and r each independently represent an integer between 0-10, with the proviso that p+q+r is at least 1, and preferably 5-20.

Because the aliphatic diamine represented by the general formula (3-1) or (4-1) includes a long chain alkyleneoxy group, the resultant polyimide exhibits superior flexibility.

The diamine constituting the polyimide may further include a third (β3) diamine other than the (β1) aromatic diamine having a benzophenone backbone and (β2) aliphatic diamine. There are no particular limitations to the third (β3) diamine; the third (β3) diamine is an aromatic diamine other than the (β1) aromatic diamine, an aliphatic diamine other than the (β2) aliphatic diamine, or alicyclic diamine. An aromatic diamine other than the (β1) aromatic diamine is preferable from the perspective of enhancing heat resistance.

Examples of the aromatic diamine other than the (β1) aromatic diamine may include m-phenylenediamine, o-phenylenediamine, p-phenylenediamine, m-aminobenzylamine, p-aminobenzylamine, bis(3-aminophenyl)sulfide, (3-aminophenyl)(4-aminophenyl)sulfide, bis(4-aminophenyl)sulfide, bis(3-aminophenyl)sulfoxide, (3-aminophenyl)(4-aminophenyl)sulfoxide, bis(3-aminophenyl)sulfone, (3-aminophenyl)(4-aminophenyl)sulfone, bis(4-aminophenyl)sulfone, 3,3'-diaminodiphenylmethane, 3,4'-diaminodiphenylmethane, 4,4'-diaminodiphenylmethane, 4,4'-diaminodiphenylether, 3,3'-diaminodiphenylether, 3,4'-diaminodiphenylether, 3,3'-dimethylbenzidine, 3,4'-dimethylbenzidine, 4,4'-dimethylbenzidine, 2,2'-bis(trifluoromethyl)-1,1'-biphenyl-4,4'-diamine, 1,3-bis(3-aminophenoxy)benzene, 1,3-bis(4-aminophenoxy)benzene, 1,3-bis(3-(3-aminophenoxy)phenoxy)benzene, 1,3-bis(3-(4-aminophenoxy)phenoxy)benzene, 1,3-bis(4-(3-aminophenoxy)phenoxy)benzene, 1,3-bis(3-(3-aminophenoxy)phenoxy)-2-methylbenzene, 1,3-bis(3-(4-aminophenoxy)phenoxy)-4-methylbenzene, 1,3-bis(4-(3-aminophenoxy)phenoxy)-2-ethylbenzene, 1,3-bis(3-(2-aminophenoxy)phenoxy)-5-sec-butylbenzene, 1,3-bis(4-(3-aminophenoxy)phenoxy)-2,5-dimethylbenzene, 1,3-bis(4-(2-amino-6-methylphenoxy)phenoxy)benzene, 1,3-bis(2-(2-amino-6-ethylphenoxy)phenoxy)benzene, 1,3-bis(2-(3-aminophenoxy)-4-methylphenoxy)benzene, 1,3-bis(2-(4-aminophenoxy)-4-tert-butylphenoxy)benzene, 1,4-bis(3-(3-aminophenoxy)phenoxy)-2,5-di-tert-butylbenzene, 1,4-bis(3-(4-aminophenoxy)phenoxy)-2,3-dimethylbenzene, 1,4-bis(3-(2-amino-3 propylphenoxy)phenoxy)benzene, 1,2-bis(3-(3-aminophenoxy)phenoxy)-4-methylbenzene, 1,2-bis(3-(4-aminophenoxy)phenoxy)-3-n-butylbenzene, 1,2-bis(3-(2-amino-3-propylphenoxy)phenoxy)benzene, 4,4'-bis(4-aminophenyl)-1,4-diisopropylbenzene, 3,4'-bis(4-aminophenyl)-1,4-diisopropylbenzene, 3,3'-bis(4-aminophenyl)-1,4-diisopropylbenzene, bis[4-(3-aminophenoxy)phenyl]methane, bis[4-(4-aminophenoxy)phenyl]methane, 1,1-bis[4-(3-aminophenoxy)phenyl]ethane, 1,1-bis[4-(4-aminophenoxy)phenyl]ethane, 1,2-bis[4-(3-aminophenoxy)phenyl]ethane, 1,2-bis[4-(4-aminophenoxy)phenyl]ethane, 2,2-bis[4-(3-aminophenoxy)phenyl]propane, 2,2-bis[4-(4-aminophenoxy)phenyl]propane, 2,2-bis[4-(3-aminophenoxy)phenyl]butane, 2,2-bis[3-(3-aminophenoxy)phenyl]-1,1,1,3,3,3-hexafluoropropane, 2,2-bis[4-(4-aminophenoxy)phenyl]-1,1,1,3,3,3-hexafluoropropane, 4,4'-bis(3-aminophenoxy)biphenyl, 4,4'-bis(4-aminophenoxy)biphenyl, 3,3'-bis(4-aminophenoxy)biphenyl, bis[4-(3-aminophenoxy)phenyl]ketone, bis[4-(4-aminophenoxy)phenyl]ketone, bis[4-(3-aminophenoxy)phenyl]sulfide, bis[4-(4-aminophenoxy)phenyl]sulfide, bis[4-(3-aminophenoxy)phenyl]sulfoxide, bis[4-(aminophenoxy)phenyl]sulfoxide, bis[4-(3-aminophenoxy)phenyl]sulfone, bis[4-(4-aminophenoxy)phenyl]sulfone, 1,4-bis[4-(3-aminophenoxy)benzoyl]benzene, 1,3-bis[4-(3-aminophenoxy)benzoyl]benzene, 4,4'-bis[3-(4-aminophenoxy)benzoyl]diphenylether, 4,4'-bis[3-(3-aminophenoxy)benzoyl]diphenylether, 4,4'-bis[4-(4-amino-α,α-dimethylbenzyl)phenoxy]benzophenone, 4,4'-bis[4-(4-amino-α,α-dimethylbenzyl)phenoxy]diphenylsulfone, bis [4-{4-(4-aminophenoxy)phenoxy}phenyl]sulfone, 1,4-bis[4-(4-aminophenoxy)-α,α-dimethylbenzyl]benzene, and 1,3-bis[4-(4-aminophenoxy)-α,α-dimethylbenzyl]benzene. Among the above examples of the aromatic diamines, 1,3-bis(3-aminophenoxy)benzene, 1,3-bis(4-aminophenoxy)benzene, 2,2-bis[4-(4-aminophenoxy)phenyl]propane, bis(3-aminophenyl)sulfone, bis(4-aminophenyl)sulfone, 4,4'-bis(4-aminophonyl)-1,4-diisopropylbenzene, 3,4'-bis(4-aminophenyl)-1,4-diisopropylbenzene, 3,3'-bis(4-aminophenyl)-1,4-diisopropylbenzene, 3,3'-bis(4-aminophenoxy)biphenyl, 2,2'-bis(trifluoromethyl)-1,1'-biphenyl-4,4'-diamine, 3,3'-dimethylbenzidine, 3,4'-dimethylbenzidine, and 4,4'-dimethylbenzidine are preferable because these diamines allow for high heat resistance without causing significant decrease in flexibility.

Examples of the aliphatic diamine other than the (β2) aliphatic diamine may include ethylene diamine, 1,3-diaminopropane, 1,4-diaminobutane, 1,5-diaminopentane, 1,6-diaminohexane, 1,7-diaminoheptane, 1,8-diaminooctane, 1,9-diaminononane, 1,10-diaminodecane, 1,11-diaminoundecane, and 1,12-diaminododecane, with ethylene diamine being preferable.

Examples of the alicyclic diamine may include cyclobutanediamine, 1,2-cyclohexanediamine, 1,3-cyclohexanediamine, 1,4-cyclohexanediamine, di(aminomethyl)cyclohexane, bis(aminomethyl)cyclohexanes other than 1,4-bis(aminomethyl)cyclohexane, diaminobicycloheptane, diaminomethylbicycloheptane (including norbornane diamines such as norbornane diamine), diaminooxybicycloheptane, diaminomethyloxybicycloheptane (including oxanorbomane diamine), isophorone diamine, diaminotricyclodecane, diaminomethyltricyclodecane, bis(aminocyclohexyl)methane (or methylene bis(cyclohexylamine)), and bis(aminocyclohexyl)isopropylidene. Among the above examples of the alicyclic diamine, norbornane diamine, 1,2-cyclohexanediamine, 1,3-cyclohexanediamine, and 1,4-cyclohexanediamine are preferable because these diamines allow for high heat resistance without causing significant decrease in flexibility.

The total amount of (α1) aromatic tetracarboxylic acid dianhydride and (β1) aromatic diamine having a benzophenone backbone is preferably 5-49 mol%, and more preferably 9-30 mol%, with respect to the total amount of the tetracarboxylic acid dianhydride and diamine constituting the polyimide. When the total amount of (α1) aromatic tetracarboxylic acid dianhydride and (β1) aromatic diamine is less than 5 mol%, the number of carbonyl groups derived from the benzophenone backbone is small. Accordingly, as will be described later, it is difficult to attain heat resistance because the carbonyl groups derived from the benzophenone backbone in one molecule cannot form sufficient hydrogen bonding with the terminal amino groups in another molecule, or because the carbonyl groups derived from the benzophenone backbone cannot form sufficient hydrogen bonding with the terminal amino groups within the same molecule.

In order to confer to the polyimide a high flexibility, the amount of the (β2) aliphatic diamine represented by the general formula (3) or (4) (i.e., the total amount of the diamine represented by the general formula (3) or (4)) is preferably at least 10 mol%, and is more preferably at least 12 mol%, with respect to the total amount of the diamine constituting the polyimide. On the other hand, in order to prevent significant reduction in the heat resistance of the polyimide, the amount of the (β2) aliphatic diamine is preferably 45 mol% or less with respect to the total amount of the diamine constituting the polyimide.

In order for the polyamide to have an amino group as the molecular terminal group, the amount of the diamine component to be reacted (b mole) may be set larger than the amount of the tetracarboxylic acid dianhydride (a mole). Specifically, the molar ratio of the tetracarboxylic acid dianhydride (a mole) to the diamine (b mole) constituting the polyimide, molar ratio a/b, is preferably 0.8 to less than 1.0, and more preferably 0.95-0.999. It is difficult to attain heat resistance when the a/b ratio is 1.0 or more, because an amino group cannot be introduced as the molecular terminal group, or because the carbonyl groups derived from the benzophenone backbone cannot form sufficient hydrogen bonding with the terminal amino groups.

The polyimide may be a random polymer or a block polymer.

The amine equivalent of the polyimide is preferably 4,000-20,000, and more preferably 4,500-18,000. The amine equivalent of the polyimide is defined as "number-average molecular weight of polyimide divided by the number of amino groups included in one molecule." Naturally, the amino groups included in one molecule include the terminal amino groups as well as other amino groups. The polyimide having an amino equivalent weight that falls within the above-described range has a high proportion of terminal amino groups in the whole polyimide, thereby allowing much hydrogen bonding to occur between the terminal amine groups and carbonyl groups of the benzophenone backbone, whereby heat resistance is increased.

The number-average molecular weight of polyimide is preferably 6.0×10³-1.0×10⁶, and more preferably 8.0×10³-4.0×10⁴. The number-average molecular weight of the polyimide is measured by gel permeation chromatography (GPC).

As previously described, the polyimide includes a benzophenone backbone derived from the (α1) aromatic tetracarboxylic acid dianhydride or (β1) aromatic diamine, and includes an amino group as the molecular terminal group. Accordingly, high heat resistance is attained due to hydrogen bonding of the carbonyl groups derived from the benzophenone backbone in one polyimide molecule with the terminal amino groups in another polyimide molecule. Moreover, because the polyimide further includes a long chain alkyleneoxy group derived from the (β2) aliphatic diamine, the polyimide has a high solvent-solubility and the resultant polyimide film exhibits high flexibility.

The polyimide resin composition of the present invention may include resins other than the above-described polyimide resin, fillers, and/or surface modifiers, where necessary.

Examples of the other resins may include epoxy compounds such as bisphenol A epoxy compounds, and bisphenol F epoxy compounds; acrylate compounds such as carboxyethylacrylate, propylenegylcolacrylate, ethoxylated phenylacrylate, and aliphatic epoxyacrylates; isocyanate compounds such as methylene bisphenyldiisocyanate (MDI), toluene diisocyanate (TDI), hexamethylene diisocyanate (HDI), and xylene diisocyanate (XDI); maleimide compounds such as 4,4'-diphenylmethanebismaleimide, 4,4'-diphenyloxybismaleimide, 4,4'-diphenylsulfonebismaleimide, p-phenylenebismaleimide, m-phenylenebismaleimide, 2,4-tolylenebismaleimide, 2,6-tolylenebismaleimide, ethylenebismaleimide, hexamethylenebismaleimide, 4,4'-(2,2'-bis(4",4"'-phenoxyphenyl)isopropylidene)bismaleimide, 4,4'-(2,2'-bis(4",4"'-phenoxyphenyl)hexafluoroisopropylidene)maleimide, 4,4'-bis(3, 5-dimethylphenylmethane maleimide, 4,4'-bis(3,5-diethylphenylmethane bismaleimide, 4,4'-bis(3-methyl-5-ethylphenyl)methane bismaleimide, 4,4'-bis(3,5-diisopropylphenyl)methane maleimide, 4,4'-dicyclohexylmethane bis maleimide, p-xylylene bismaleimide, m-xylylene bismaleimide, 1,3-dimethylenecyclohexane bismaleimide, 1,4-dimethylenecyclohexane bismaleimide, and aminophenoxybenzene-bismaleimide (APB-BMI); and nadimide compounds such as alkenyl-substituted nadimides. For example, a photocurable resin or photocurable agent such as an acrylate compound may be included in the polyimide resin composition when attempting to provide photosensitivity to the polyimide resin composition.

The polyimide resin composition may also contain a flame retardant. There are no particular limitations to the flame retardant; for example, halogenated flame retardants, inorganic flame retardants, and phosphorus flame retardants may be employed. A single flame retardant may be employed or a blend of two or more different flame retardants may be employed. Organic compounds that contain chlorine and compounds that contain bromine may be exemplified as the halogenated flame retardants. Specifically, pentabromodiphenylether, octabromodiphenylether, decabromodiphenylether, tetrabromobisphenyl A, and hexabromocyclodecanetetrabromobispenol A may be exemplified. Antimony compounds and metal hydroxides may be exemplified as the inorganic flame retardants. Antimony trioxide and antimony pentoxide may be exemplified as the antimony compounds. Aluminum hydroxide and magnesium hydoxide may be exemplified as the metal hydroxides. Phosphazene, phosphine, phosphine oxide, and phosphoric acid ester may be exemplified as the phosphorus flame retardants. The amount of flame retardant to be added is not particularly limited, and thus may appropriately determined in accordance with the type of flame retardant to be employed. In general, the flame retardant is preferably present in an amount of 5 parts by mass to 50 parts by mass with respect to 100 parts by mass of the polyimide resin.

In order to increase the heat resistance or thermal conductivity of the polyimide resin composition, the filler is preferably an inorganic filler. The inorganic filler may be, for example, a heat-dissipating filler, an electroconductive filler, or a magnetic filler. The heat-dissipating filler may be formed of a material that has an electrical insulating property and a high heat-dissipating property. Examples of the heat-dissipating filler may include boron nitride, aluminum nitride, aluminum oxide, hydrated aluminum oxide, silicon oxide, silicon nitride, silicon carbide, diamond, hydroxyapatite, and barium titanate, with boron nitride being preferable. The heat-dissipating filler content with respect to the total volume of the polyimide resin composition is preferably 20-60vol%, with the upper limit being more preferably 50vol%. The polyimide resin composition exhibits a superior heat-dissipating property when the volume of heat-dissipating filler falls within the above-described range.

The electrocunductive filler may be formed of a material that has an electroconductive property. Examples of the electroconductive filler may include metal powders, metal flakes, metal ribbons, metal fibers, metal oxides, fillers covered with conductive material, carbon powders, graphites, carbon fibers, carbon flakes, and flakey carbon. The magnetic filler may be formed of a material having a magnetic property. Examples of the magnetic filler may include sendusts, permalloys, amorphous alloys, stainless steel, MnZn ferrites, and NiZn ferrites. The total volume of the electroconductive filler and magnetic filler with respect to the total volume of the polyimide resin composition is preferably 20-90vol%, and more preferably 30-80vol%. The polyimide resin composition exhibits a superior electroconductive property when the total volume of the electroconductive filler and magnetic filler falls within the above-described range.

Examples of the surface modifier may include silane coupling agents. The surface modifier may be added for the purpose of treating the filler's surface. This improves the compatibility of the filler with polyimide allowing for control of aggregation and/or dispersed state of filler particles.

The polyimide resin composition of the present invention may be provided as a varnish or as a film.

When the polyimide resin composition is a varnish, the polyimide resin composition may further contain a solvent when necessary. Examples of the solvent may include NN-dimethylformamide, N,N-dimethylacetamide, N,N-diethylformamide, N,N-diethylacetamide, N,N-dimethylmethoxyacetamide, dimethylsulfoxide, hexamethylphosphoramide, N-methyl-2-pyrrolidone, dimethylsulfone, 1,3,5-trimethylbenzene, 1,2,4-trimethylbenzene, mixed solvents of two or more of the foregoing, and mixed solvents of one or more of the foregoing and one or more solvents selected from benzene, toluene, xylene, benzonitrile, dioxane, cyclohexane, and other solvents. The concentration of resin solid in the polyimide varnish is preferably 5-50wt%, and more preferably 10-30wt%, from the perspective of enhancing coatability.

A measured viscosity at 25°C by an E-type viscometer of a polyimide solution obtained by dispersing 20wt% of polyimide in a mixed solvent of NMP and trimethylbenzene is preferably 5.0×10²-1.0×10⁶ mPa·s, and more preferably 1.0×10³-5.0×10⁴ mPa·s, from the perspective of coatability.

The polyimide varnish is prepared by formulating an acid anhydride component and a diamine component in a solvent, obtaining a polyamic acid by addition polymerization of the two components, and imidizing the polyamic acid by dehydration condensation. The acid anhydride component and diamine component formulated may be any of the components described above.

The polyimide is soluble in polar solvent. Accordingly, the polyimide resin composition of the present invention may be formulated into a polyimide varnish in which the polyimide is dissolved in any of the above-described solvents. A polyimide film is then manufactured by applying the polyimide resin composition of the present invention on a substrate, followed by drying. In this way it is possible to eliminate the high-temperature imidization of a coated film of the polyimide resin composition of the present invention, making it possible to form a polyimide layer even on low-heat resistant substrates by coating techniques.

As used herein, the phrase "polyamide is soluble in polar solvent" means that in a solution containing 5wt% polyimide as a solute in N-methyl-2-pyrrolidone as a solvent, there occurs no polyimide precipitation and/or gelation. Preferably, no polyimide precipitation and/or gelation occur even in the solution containing 50wt% polyimide. Precipitation and gelation of the polyimide are confirmed by visual observation.

When the polyimide resin composition is a film, the thickness of the film may be 2-200 µm.

The glass transition temperature of the film formed of the polyimide resin composition is preferably 120°C to less than 260°C, and more preferably 130°C-210°C, In cases where the glass transition temperature of the polyimide resin composition is 260°C or above, for example, when the polyimide resin composition is used as a film-shaped adhesive it does not readily adhere at low temperatures (i.e., thermocompression bonding).

The glass transition temperature of a film formed of the polyimide resin composition may be measured by the following procedure. Specifically, a polyimide film having a thickness of 50 µm is provided. A temperature dispersion of the dynamic viscoelasticity of the film is measured in tension mode at a measurement frequency of 1 Hz, thereby measuring a storage modulus of elasticity E' and a loss modulus of elasticity E". In addition, a peak value of the obtained loss tangent tan δ = E"/ E' is defined as "glass transition temperature."

The storage modulus of elasticity (at glass transition temperature + 30°C) of the film formed of the polyimide resin composition is preferably at least 1.0×10⁵ Pa, and more preferably at least 5.0×10⁶ Pa. The storage modulus of elasticity is found by identifying the storage modulus of elasticity at a glass transition temperature + 30°C from the profile of solid viscoelasticity obtained by the above-described measurement of glass transition temperature. Moreover, the storage modulus of elasticity E' at 180°C of the film formed of the polyimide resin composition is preferably 1.0×10⁵ Pa or more, and more preferably 1.0×10⁶ Pa or more. The storage modulus of elasticity E' at 180°C is also found from the profile of solid viscoelasticity obtained by the above-described measurement of glass transition temperature.

The elongation rate of a 50 µm-thick film formed of the polyimide resin composition at the time of tensile fracture at 23°C is preferably 50% or more, and more preferably 80% or more. Such a polyimide resin composition is suitable in applications where flexibility is required. The elongation rate of the film at the time of tensile fracture is defined as [(length of sample film at the time of fracture - original length of sample film)/(original length of sample film)] for a film which is formed of the polyimide resin composition which measures 10 mm in width 140 mm in length and which has been elongated along its length at 23°C and at a rate of 50 mm/min using a material testing machine TENSILON.

As previously mentioned, the polyimide contained in the polyimide resin composition of the present invention includes a benzophenone backbone derived from the (α1) aromatic tetracarboxylic acid dianhydride or (β1) aromatic diamine, and includes an amino group at its molecular terminal group. Accordingly, the film obtained from the polyimide resin composition exhibits high heat resistance due to the hydrogen bonding of the carbonyl groups derived from the benzophenone backbone in one polyimide molecule and with the terminal amino groups of another polyimide molecule. Moreover, the polyimide contained in the polyimide resin composition of the present invention further includes a long chain alkyleneoxy group derived from the (β2) aliphatic diamine. Accordingly, a polyimide layer or film obtained from the polyimide resin composition of the present invention exhibits high heat resistance and high flexibility.

### 2. Application of polyimide resin composition

The film obtained from the polyimide resin composition of the present invention exhibits high heat resistance and high flexibility. Accordingly, the polyimide resin composition of the present invention is specifically intended for applications where heat resistance and flexibility are required. For example, the polyimide resin composition of the present invention may be used as an adhesive, a sealant, an insulating material, a substrate material or a protective material in electronic circuit board members, semiconductor devices, lithium-ion battery members, solar cell members, fuel cell members, motor winding, engine peripheral members, coatings, optical parts, heat-dissipating substrates, electromagnetic wave shielding substrates, surge parts, and/or the like.

Namely, a laminate may be provided that has a substrate and a resin layer including the polyimide resin composition of the present invention disposed on the substrate. Although the type of substrate depends on the application of the laminate, the substrate may be composed of silicon, ceramic, metal or the like. Examples of the metal may include silicon, copper, aluminum, SUS, iron, magnesium, nickel, and aluminum oxide. Examples of the resin may include urethane resins, epoxy resins, acrylic resins, polyimide resins, PET resins, polyamide resins, and polyamide-imide resins.

The above-described laminate may be prepared by applying the polyimide resin composition of the present invention on the substrate, and drying the polyimide resin composition to form a resin layer formed of the polyimide resin composition, or may be prepared by thermocompression bonding of the film formed of the polyimide resin composition of the present invention to the film, to form a resin layer formed of the polyimide resin composition. When forming the resin layer formed of the polyimide resin composition by applying and drying the polyimide resin composition of the present invention, the drying temperature of the coated film is preferably 250°C or below.

### Electronic circuit board member

The polyimide resin composition of the present invention may be configured as an insulating substrate or adhesive material for a circuit board, particularly for a flexible circuit board. For example, the flexible circuit board may include a metal foil (substrate) and an insulating layer formed of the polyimide resin composition of the present invention disposed on the metal foil. Alternatively, the flexible circuit board may include an insulating resin film (substrate), an adhesive layer formed of the polyimide resin composition of the present invention, and a metal foil.

### Semiconductor device

The polyimide resin composition of the present invention may be an adhesive for bonding one semiconductor chip to another or bonding a semiconductor chip to the substrate; a protective material for protecting a circuit of a semiconductor chip; and an encapsulating material (sealant) for encapsulating therein a semiconductor chip. When the polyimide resin composition of the present invention further includes an inorganic filler, the polyimide resin composition may be used an adhesive that exhibits a superior heat-dissipating property.

Namely, the semiconductor device of the present invention includes a semiconductor chip (substrate) and a resin layer formed of the polyimide resin composition of the present invention disposed on at least one side of the semiconductor substrate. The semiconductor chip may include a diode, a transistor and an integrated circuit (IC), as well as a power element and other elements. The resin layer formed of the polyimide resin composition of the present invention may be disposed on a surface where the terminal of the semiconductor chip is formed (terminal formation surface), or may be disposed on a surface remote from the terminal formation surface.

The thickness of the layer formed of the polyimide resin composition, e.g., when it is configured as an adhesive layer, is preferably 1-100 µm. When providing a circuit protective layer of the polyimide resin composition, a thickness of 2-200 µm is preferable.

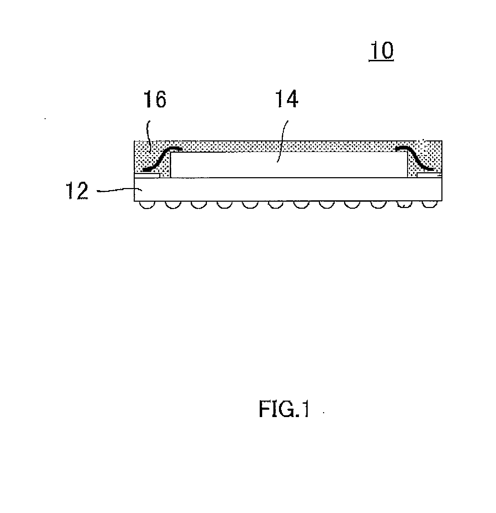

The polyimide resin composition of the present invention will now be described by way of an encapsulating material for encapsulating therein a semiconductor. FIG. 1 is a schematic view showing an example of ball grid array (BGA) packaging. As shown in FIG. 1, BGA packaging 10 includes substrate 12 and semiconductor chip 14 disposed on a surface of substrate 12 remote from the ball grid array. BGA packaging 10 is sealed by sealing layer 16 on the surface of semiconductor chip 14 (substrate). Sealing layer 16 may be the polyimide resin composition of the present invention.

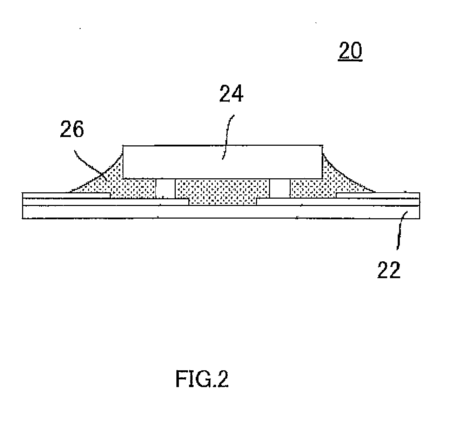

The polyimide resin composition of the present invention will now be described by way of a filler material for filling a gap formed between a semiconductor chip and the substrate. FIG. 2 is a schematic view showing an example of chip-on-film (COP) packaging. As shown in FIG. 2, COP packaging 20 includes film substrate 22 and semiconductor chip 24 disposed on one surface of film substrate 22. The gap between semiconductor chip 24 and film substrate 22 (substrate) is sealed by underfill layer 26. Underfill layer 26 may be the polyimide resin composition of the present invention.

### Solar cell member

The polyimide resin composition of the present invention may be configured as a substrate or a frame-shaped sealant in a solar cell module, or as an insulting protective film to be disposed on the surface of an ITO electrode of an organic thin-film solar cell or a dye-sensitized solar cell. Specifically, a solar cell module usually includes solar cells, a pair of substrates (protective members) that sandwich the solar cells, and a sealing layer filling a space between at least one of the substrate and the solar cell, and further seals the periphery of the solar cell module with a sealant. The sealant to be disposed on the substrate (protective member) or in frame shape may be the polyimide resin composition of the present invention.

### Lithium-ion battery member

The polyimide resin composition of the present invention may be configured as a binder for use in a lithium-ion battery for fixing electrode active substances on metal foils, particularly as a binder for fixing a large-capacity negative electrode active substance (e.g., silicon particles) on a negative electrode plate (foil), or as a separator.

Namely, the lithium-ion secondary cell has a metal foil (collector foil), an electrode active substance disposed on the metal foil, and an active substance layer including a binder. The binder in the active substance layer may be the polyimide resin composition of the present invention.

As the electrode active substances experience repeated cycles of adsorption and discharge of lithium ions during the charge/discharge cycles in the lithium-ion secondary cell, expansion and shrinkage of the electrode active substance is increased facilitating separation (fall) of the electrode active substance. The polyimide resin composition of the present invention exhibits adhesiveness capable of withstanding the expansion or shrinkage of the electrode active substance accompanied by charging and discharging, as well as heat resistance. Therefore, separation of the electrode active substance may be limited.

The separator of the lithium-ion secondary cell may contain the polyimide resin composition of the present invention. For example, the separator of the lithium-ion secondary cell may be woven from fibers formed of the polyimide resin composition. Accordingly, the separator containing the polyimide resin composition of the present invention exhibits higher heat resistance than conventional olefin-based separators.

### Heat-dissipating substrate

The polyimide resin composition of the present invention may be configured as a heat-dissipating substrate for cooling semiconductor devices, home appliances, personal computers, motors, mobile devices, and other devices. The conventional heat-dissipating substrates are formed of silicone resin, epoxy resin, acryl resin and/or the like, but they are not only insufficient in heat resistance, flexibility and insulative property, but also contain volatile organic compounds (VOCs). In contrast, when the polyimide resin composition of the present invention is configured as a heat-dissipating substrate, heat resistance, flexibility and insulative property can be improved, and moreover, the amount of VOCs can be reduced. When the polyimide resin composition of the present invention is configured as a heat-dissipating substrate, the polyimide resin composition preferably contains 20-60vol% heat-dissipating filler with respect to the total volume of the polyimide resin composition.

### Electromagnetic shielding substrate

The polyimide resin composition of the present invention may be configured as an electromagnetic shielding substrate that shields external electromagnetic waves that have impacts on semiconductor devices, home appliances, personal computers, transportation systems such as automobiles, mobile devices and other devices, or that shields internal electromagnetic waves generated from these devices The conventional electromagnetic shielding substrates are made of silicone resin, epoxy resin, acryl resins and/or the like, but they are not only insufficient in heat resistance, flexibility and insulative property, but also contain volatile organic compounds (VOCs). In contrast, when the polyimide resin composition of the present invention is configured as an electromagnetic shielding substrate, heat resistance and flexibility can be improved, and moreover, the amount of VOCs can be reduced. When the polyimide resin composition of the present invention is configured as an electromagnetic shielding substrate, the polyimide resin composition preferably contains 20-90vol% electroconductive filler and/or magnetic filler with respect to the total volume of the polyimide resin composition.

### Adhesives for surge parts and sealants for surge parts

The polyimide resin composition of the present invention may be configured as an adhesive for surge parts (surge absorbers) or as a sealant for surge parts to protect home appliances, personal computers, transportation systems such as automobiles, mobile devices, power sources, servers, telephones and other devices from the impact of an abnormal current or voltage. The conventional adhesives or sealants for surge parts are welding fluxes such as silver-solder, but they require a high-temperature process, and the cost of the materials thereof is high. Moreover, when the above-described adhesive or sealant is employed for resin adhesion, not only withstand voltage and heat resistance are insufficient, but also volatile organic compounds (VOCs) are included therein. On the other hand, when the polyimide resin composition of the present invention is used as the adhesive or sealant, the surge part may be adhered or sealed at low temperatures, and the withstand voltage and heat resistance are sufficient. In addition, the amount of VOCs can be reduced, which is also preferable from the perspective of cost.

### Adhesive application for semiconductor manufacturing devices

The polyimide resin composition of the present invention is suitably used as an adhesive employed in semiconductor manufacturing devices, particularly as an adhesive for electrostatic chucks. Conventionally, gum adhesives such as butyl gum with a stress-relaxation property, and epoxy or polyimide adhesives with heat resistance have been used as adhesives applied between a ceramic electrostatic chuck and an aluminum plate electrode. However, when the semiconductor manufacturing temperature is high, the heat resistance of the gum adhesive is insufficient. On the other hand, the ceramic electrostatic chuck may break as a result of insufficient stress-relaxation in the epoxy- or polyimide adhesives. In addition, while it is necessary to thin an adhesive layer in order to increase the heat-dissipation of the electrostatic chuck, it has been pointed out that gum adhesives and epoxy adhesives other than polyimide cause insulation breakdown due to their low insulating property. With regard to the polyimide resin composition of the present invention, because heat resistance and flexibility are considered compatible with insulating property and because of its thermoplastic property, the polyimide resin composition of the present invention may be used as an adhesive without any modifications thereto. Accordingly, the polyimide resin composition is suitable in the present application.

### Application for dental materials

The polyimide resin composition of the present invention can be suitably employed as a surface coating material or adhesive for dental materials such as artificial teeth or dentures. Acrylic coating materials have heretofore been employed as surface coatings of artificial teeth. However, abrasion resistance and rub resistance are insufficient as an artificial tooth. The polyimide resin composition of the present invention is superior in mechanical strength and also in abrasion resistance, and thus the polyimide resin composition is preferred as a surface coating or surrounding adhesive of an artificial tooth when mixed with a white filler and/or the like.

### Examples

The acid anhydrides and diamines used in Examples and Comparative Examples are given below.
(1) Acid dianhydrides
   1) (α1) aromatic tetracarboxylic acid dianhydride having a benzophenone backbone represented by general formula (1)
      BTDA: 3,3',4,4'-benzophenone tetracarboxylic acid dianhydride
   2) (α2) additional tetracarboxylic acid dianhydride
      s-BPDA: 3,3',4,4'-biphenyltetracarboxylic acid dianhydride (manufactured by JFE Chemical Corporation)
      PMDA: pyromellitic dianhydride
      ODPA: oxydiphthalic dianhydride

(2) Diamines
   1) (β2) aliphatic diamine represented by general formula (3) or (4) 14EL: polytetramethyleneoxide di-p-aminobenzoate (ELASMER-1000; manufactured by IHARA CHEMICAL INDUSTRY CO., LTD.) 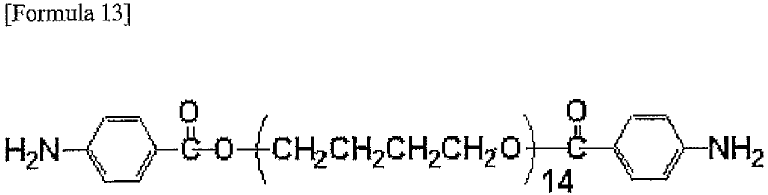 XTJ-542: polyetheramine represented by the following formula (product name: JEFFAMINE; manufactured by Huntsman International, LLC.) 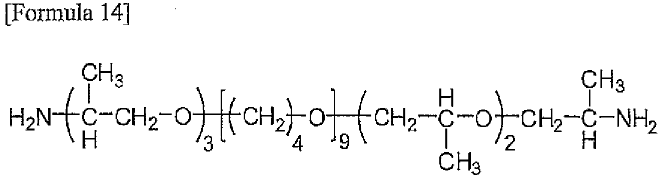 D-2000: polyoxyproylenediamine (manufactured by MITSUI FINE CHEMICALS, INC.)
   2) (β3) diamine
      APB; 1,3,-bis(3-aminophenoxy)benzene (manufactured by MITSUI CHEMICALS, INC.)
      p-BAPP: 2,2-bis(4-(4-aminophenoxy)phenyl)propane
      1-Si: 1,3-bis(3-aminopropyl)tetramethyldisiloxane
      m-BP: 4,4'-bis(3-aminophenoxy)biphenyl

### (Example 1)

### Preparation of polyimide varnish

Two acid dianhydrides (s-BPDA and BTDA) and three diamines (APB, 14EL and XTJ-542) were mixed at a molar ratio of s-BPDA:BTDA:APB:14EL:XTJ-542 = 0.79:0.2:0.8:0.1:0.1 in a 7:3 mixture solvent of N-methylpyrrolidone (NMP) and mesitylene. The compound obtained was stirred for no less than four hours in a flask that can be purged with dry nitrogen gas, and a polyamic acid solution was obtained that contained 20-25wt% resin solid. The polyamic acid solution obtained was sufficiently stirred, the reaction system was heated to 180°C while stirring in a flask attached to a Dean-Stark apparatus, and the water generated by the dehydration reaction was distilled out of the system to afford a polyimide varnish.

### 1) Varnish stability

The prepared polyimide varnish was placed in a small bottle and stored for three months in a refrigerator that was set to 3°C. The appearance of the polyimide varnish was visually observed every several weeks. Specifically, the occurrence of resin precipitation or gelation was visually observed. Then, varnish stability was evaluated based on the following criteria.
○: Precipitation or gelation of resin did not occur, even after three months had passed.
□: Precipitation or gelation of resin occurred within one month to three months.
×: Precipitation or gelation of resin occurred within one month.

### Preparation of film

The polyimide varnish obtained was applied at a speed of 10 mm/sec on a release-treated PET film, and the solvent was removed by drying for 10 minutes at 200°C. The dried film obtained from the polyimide was peeled from the PET film using a tweezers to afford a polyimide film having a thickness of 50 µm.

### 2) Heat resistance

As a sample film, the prepared polyimide film was cut into a strip shape having a width of 10 mm by a length of 100 mm. An observation was made on whether or not the sample film melted after floating for a predetermined time in a solder bath heated to a predetermined temperature. Then, the heat resistance of the sample film was evaluated based on the following criteria.
□: No melting even after 30 seconds at 280°C.
○: Although slight melting occurred after 60 seconds at 260°C, the level of melting was such that the film shape was retained and the film could be lifted.
×: Melting occurred after 60 seconds at 260°C.

### 3) Glass transition temperature; and 4) Storage modulus of elasticity

Storage modulus of elasticity E' and loss modulus of elasticity E" of the prepared polyimide film were measured by measuring a temperature dispersion of solid viscoelasticity using RSA-II (manufactured by TA Instruments) in tension mode at a measuring frequency of 1 Hz. The glass transition temperature was then found based on the peak value of loss tangent tan δ = E"/ E'.

Moreover, the storage modulus of elasticity E' of the polyimide film at a temperature 30°C higher than the glass transition temperature was evaluated based on the following criteria.
○: Storage modulus of elasticity E' is no less than 1.0 × 10⁵ Pa.
×: Storage modulus of elasticity E' is less than 1.0 × 10⁵ Pa.
In addition, the storage modulus of elasticity at 180°C was specified. The storage modulus of elasticity E' at 180°C was also evaluated based on the following criteria.
○: Storage modulus of elasticity E' is no less than 1.0 × 10⁵ Pa.
×: Storage modulus of elasticity E' is less than 1.0 × 10⁵ Pa.

### 5) Elongation rate at the time of tensile fracture and fracture strength

The prepared polyimide film was cut to a width of 10 mm by a length of 140 mm, to prepare a sample film. Using a material testing machine TENSILON, a portion of the sample film that is 10 mm wide and 140 mm length was elongated along its length at a speed of 50 mm/min at 23°C while clamping both 20 mm ends as the gripping tabs. "Elongation rate at the time of tensile fracture" was then found by calculating [(length of sample film at time of fracture - original length of sample film)/(original length of sample film)]. In addition, the elongation rate at the time of tensile fracture of the sample film was evaluated based on the following criteria.
○: Elongation rate at the time of tensile fracture is no less than 50%.
×: Elongation rate at the time of tensile fracture is less than 50%.
Moreover, the elongation rate at the time of tensile fracture of the sample film was defined as the fracture strength.

### (Example 2)

A polyimide varnish and a polyimide film were prepared and evaluated in a manner similar to that in Example 1 except that two acid dianhydrides (s-BPDA and BTDA) and three diamines (APB, 14EL and XTJ-542) were mixed at a molar ratio of s-BPDA:BTDA:APB:14EL:XTJ-542 = 0.39:0.6:0.8:0.1:0.1 in a 7:3 mixture solvent of NMP and mesitylene.

### (Example 3)

A polyimide varnish and a polyimide film were prepared and evaluated in a manner similar to that in Example 1 except that two acid dianhydrides (s-BPDA and BTDA) and three diamines (p-BAPP, 14EL and XTJ-542) were mixed at a molar ratio of s-BPDA:BTDA:p-BAPP:14EL:XTJ-542 = 0.78:0.2:0.8:0.1:0.1 in a 7:3 mixture solvent of NMP and mesitylene.

### (Example 4)

A polyimide varnish and a polyimide film were prepared and evaluated in a manner similar to that in Example 1 except that two acid dianhydrides (s-BPDA and BTDA) and three diamines (p-BAPP, 14EL and XTJ-542) were mixed at a molar ratio of s-BPDA:BTDA:p-BAPP:14EL:XTJ-542 = 0.59:0.4:0.7:0.1:0.2 in a mixture 7:3 solvent of NMP and mesitylene.

### (Example 5)

A polyimide varnish and a polyimide film were prepared and evaluated in a manner similar to that in Example 1 except that two acid dianhydrides (s-BPDA and BTDA) and two diamines (APB and 14EL) were mixed at a molar ratio of s-BPDA:BTDA:APB:14EL = 0.79:0.2:0.8:0.2 in a 7:3 mixture solvent of NMP and mesitylene.

### (Example 6)

A polyimide varnish and a polyimide film were prepared and evaluated in a manner similar to that in Example 1 except that two acid dianhydrides (s-BPDA and BTDA) and two diamines (p-BAPP and XTJ-542) were mixed at a molar ratio of s-BPDA:BTDA:p-BAPP:XTJ-542 = 0.79:0.2:0.9:0.1 in a 7:3 mixture solvent of NMP and mesitylene.

### (Example 7)

A polyimide varnish and a polyimide film were prepared and evaluated in a manner similar to that in Example 1 except that two acid dianhydrides (s-BPDA and BTDA) and two diamines (p-BAPP and D-2000) were mixed at a molar ratio of s-BPDA:BTDA:p-BAPP:D-2000 = 0.59:0.4:0.8:0.2 in a 7:3 mixture solvent of NMP and mesitylene.

### (Example 8)

A polyimide varnish and a polyimide film were prepared and evaluated in a manner similar to that in Example 1 except that two acid dianhydrides (s-BPDA and BTDA) and four diamines (p-BAPP, m-BP, 14EL and XT-J542) were mixed at a molar ratio of s-BPDA:BTDA:p-BAPP:m-BP:14EL:XTJ-542 = 0.79:0.2:0.2:0.6:0.1:0.1 in a 7:3 mixture solvent of NMP and mesitylene.

### (Comparative Example 1)

A polyimide varnish was prepared in a manner similar to that in Example 1 except that one acid dianhydride (PMDA) and one diamine (APB) were mixed at a molar ratio of PMDA:APB = 1.0:1.0. However, film preparation failed due to low varnish stability.

### (Comparative Example 2)

A polyimide varnish was prepared in a manner similar to that in Example 1 except that one acid dianhydride (BTDA) and one diamine (APB) were mixed at a molar ratio of BTDA:APB = 1.0:1.0. However, film preparation failed due to low varnish stability.

### (Comparative Example 3)

A polyimide varnish and a polyimide film were prepared and evaluated in a manner similar to that in Example 1 except that three acid dianhydrides (s-BPDA, BTDA and ODPA) and one diamine (APB) were mixed at a molar ratio of s-BPDA: BTDA:ODPA:APB = 0.68:0.2:0.1:1.0.

### (Comparative Example 4)

A polyimide varnish and a polyimide film were prepared and evaluated in a manner similar to that in Example 1 except that one acid dianhydride (s-BPDA) and four diamines (APB, 14EL, XTJ-542 and 1-Si) were mixed at a molar ratio of s-BPDA:APB:14EL:XTJ-542:1-Si = 0.99:0.5:0.2:0.2:0.1.

### (Comparative Example 5).

A polyimide varnish and a polyimide film were prepared and evaluated in a manner similar to that in Example 1 except that two acid dianhydrides (s-BPDA and BTDA) and two diamines (APB and 1-Si) were mixed at a molar ratio of s-BPDA: BTDA:APB:1-Si = 0.79:0.2:0.85:0.15.

### (Comparative Example 6)

A polyimide varnish was prepared in a manner similar to that in Example 1 except that one acid dianhydride (s-BPDA) and one diamine (m-BP) were mixed at a molar ratio of s-BPDA:m-BP = 1:1 in a 7:3 mixture solvent of NMP and mesitylene. However, film preparation failed due to low varnish stability.

Table 1 shows the evaluation results of Examples 1-8 and Comparative Examples 1-6. The amine equivalent of the polyimide shown in Table 1 was determined by measuring the number-average molecular weight of the polyimide, and dividing the obtained number-average molecular weight of the polyimide by the number of amino groups in one molecule. Moreover, the total amount of monomers having a benzophenone backbone was found as the ratio of the total number of moles of monomers having a benzophenone backbone (dianhydride or diamine) with respect to the total number of moles of the dianhydride and the diamine constituting the polyimide.

**Table1**

| | | | Example | | | | | | | | Comparative Example | | | | | |
|---|---|---|---|---|---|---|---|---|---|---|---|---|---|---|---|---|
| | | | 1 | 2 | 3 | 4 | 5 | 6 | 7 | 8 | 1 | 2 | 3 | 4 | 5 | 6 |
| Composition | Acid dianhydride | s-BPDA | 0.79 | 0.39 | 0.78 | 0.59 | 0.79 | 0.79 | 0.59 | 0.79 | | | 0.68 | 0.99 | 0.79 | 1.0 |
| | | BTDA | 0.2 | 0.6 | 0.2 | 0.4 | 0.2 | 0.2 | 0.4 | 02 | | 1.0 | 0.2 | | 02 | |
| | | PMDA | | | | | | | | | 1.0 | | | | | |
| | | ODPA | | | | | | | | | | | 0.1 | | | |
| | Diamine | APB | 0.8 | 0.8 | | | 0.8 | | | | 1.0 | 1.0 | 1.0 | 0.5 | 0.85 | |
| | | p-BAPP | | | 0.8 | 0.7 | | 0.7 | 0.8 | 0.2 | | | | | | |
| | | m-BP | | | | | | | | 0.6 | | | | | | 1.0 |
| | | 14EL | 0.1 | 0.1 | 0.1 | 0.1 | 0.2 | 0.2 | | 0.1 | | | | 0.2 | | |
| | | XTJ-542 | 0.1 | 0.1 | 0.1 | 0.2 | | 0.1 | | 0.1 | | | | 0.2 | | |
| | | D-2000 | | | | | | | 0.2 | | | | | | | |
| | | 1-Si | | | | | | | | | | | | 0.1 | 0.15 | |
| | Acid/Amine equivalent ratio | | 0.99 | 0.99 | 0.98 | 0.99 | 0.99 | 0.99 | 0.99 | 0.99 | 1.00 | 1.00 | 0.98 | 0.99 | 0.99 | 1.00 |
| | Amine equivalent weight of polyimide | | 14000 | 13000 | 14000 | 15000 | 13000 | 16000 | 13000 | 16000 | Measurement not possible | Measurement not possible | 25000 | 12000 | 23000 | Measurement not possible |
| | Amount of monomer having a benzophenone backbone (mol%) | | 10 | 30 | 10 | 20 | 10 | 10 | 20 | 10 | 0 | 50 | 10 | 0 | 10 | 0 |
| Evaluation | Varnish stability | | ○ | ○ | ○ | ○ | ○ | ○ | ○ | ○ | × | × | ○ | ○ | ○ | × |
| | Heat resistance (in solder bath) | | ○ | ⊚ | ⊚ | ⊚ | ○ | ⊚ | ○ | ⊚ | - | - | ⊚ | × | ○ | - |
| | | | 260°C × 60sec | 280°C × 30sec | 280°C × 30sec | 280°C × 30sec | 260°C × 60sec | 280°C × 30sec | 260°C × 60sec | 280°C × 30sec | | | 280°C × 30sec | | 260°C × 60sec | |
| | Glass transition temperature (°C) | | 150 | 140 | 170 | 160 | 155 | 210 | 125 | 165 | - | - | 200 | 70 | 170 | - |
| | Storage modulus of elasticity (Tg + 30°C) | | ○ | ○ | ○ | ○ | ○ | ○ | ○ | ○ | - | - | ○ | × | ○ | - |
| | Storage modulus of elasticity (180°C) | | ○ | ○ | ○ | ○ | ○ | ○ | ○ | ○ | - | - | ○ | × | ○ | - |
| | Elongation rate at time of tensile fracture (%) | | 250 | 250 | 160 | 190 | 230 | 80 | 180 | 200 | - | - | 7 | 450 | 10 | - |
| | | | (○) | (○) | (○) | (○) | (○) | (○) | (○) | (○) | | | (×) | (○) | (×) | |
| | Fracture strength (MPa) | | 67 | 70 | 90 | 75 | 75 | 70 | 38 | 78 | | | 90 | 60 | 90 | |

As shown in Table 1, the polyimides of Examples 1-8, which have a benzophenone backbone and a long chain alkyleneoxy group derived from an aliphatic diamine and which have an amine equivalent within a predetermined range, were found to exhibit high varnish stability, as well as superior heat resistance and elongation rate in a film obtained from the polyimide.

In contrast, the polyimides of Comparative Examples 1, 2 and 6, which lacked a long chain alkyleneoxy group derived from an aliphatic diamine, exhibited low varnish stability, and thus film preparation failed. On the other hand, the polyimide of Comparative Example 4, which had a long chain alkyleneoxy group derived from an aliphatic diamine but lacked a benzophenone backbone, was found to exhibit high varnish stability but provide low heat resistance in a film obtained therefrom. Moreover, the polyimide of Comparative Example 3, which had a benzophenone backbone but lacked a long chain alkyleneoxy group derived from an aliphatic diamine, was found to exhibit high varnish stability and provide superior heat resistance to a film obtained therefrom, but the film exhibited low elongation rate. In addition, the polyimide of Comparative Example 5, which lacked a long chain alkyleneoxy group derived from an aliphatic diamine but had an alkylene group derived from polymethylene siloxane, was found to exhibit excellent varnish stability and provide excellent heat resistance to a film obtained therefrom, but the film exhibited low elongation rate.

### Industrial Applicability

A polyimide resin composition of the present invention is superior in solvent-solubility, and exhibits high viscoelasticity and high flexibility at high temperatures. Accordingly, the polyimide resin composition of the present invention is suitable as an adhesive for various fields in which high heat resistance and flexibility are required, e.g., the polyimide resin composition is suitable as an adhesive for electronic circuit board members, semiconductor devices, lithium-ion battery members, solar cell members and the like.

### Reference Signs List

10 BGA packaging
12, 22 substrate
14, 24 semiconductor chip
16 sealing layer
20 COF packaging
26 underfill layer

## Claims

1. A polyimide resin composition comprising:
a polyimide including a polycondensation unit of a tetracarboxylic acid dianhydride and a diamine, wherein
the tetracarboxylic acid dianhydride constituting the polyimide includes an (α1) aromatic tetracarboxylic acid dianhydride having a benzophenone backbone represented by the following general formula (1), or the diamine constituting the polyimide includes an (β1) aromatic diamine having a benzophenone backbone represented by the following general formula (2),
the diamine constituting the polyimide includes an (β2) aliphatic diamine represented by the following general formula (3) or (4),
a total amount of the (α1) aromatic tetracarboxylic acid dianhydride having the benzophenone backbone represented by the general formula (1) and the (β1) aromatic diamine having the benzophenone backbone represented by the general formula (2) is 5 to 49 mol% with respect to a total amount of the tetracarboxylic acid dianhydride and the diamine constituting the polyimide, and
an amine equivalent of the polyimide is 4,000 to 20,000, 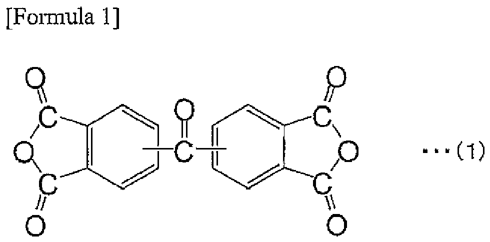 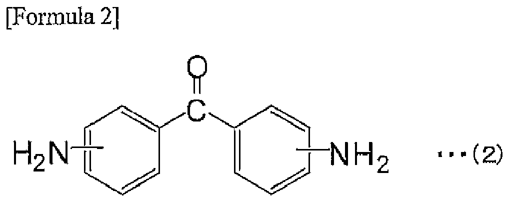 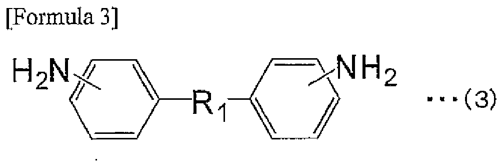
wherein in the formula (3), R₁ is an aliphatic chain including a main chain including at least one of C, N and O, a total number of atoms constituting the main chain is 7 to 500; and the aliphatic chain may also include a side chain including at least one C, N, H or O atom, and a total number of atoms constituting the side chain is 10 or less,
[Formula 4]
H₂N-R₂-NH₂ (4)
wherein in the formula (4), R₂ is an aliphatic chain including a main chain including at least one of C, N and O, a total number of atoms constituting the main chain is 5 to 500; and the aliphatic chain may also include a side chain including at least one C, N, H or O atom, and a total number of atoms constituting the side chain is 10 or less.

2. The polyimide resin composition according to claim 1, wherein a total number of moles of the tetracarboxylic acid dianhydride constituting the polyimide is 0.95 to 0.999 with respect to a total number of moles of the diamine constituting the polymide.

3. The polyimide resin composition according to claim 1, further comprising a solvent, wherein the polyimide is dissolved into the solvent.

4. The polyimide resin composition according to claim 1, wherein R₁ in the general formula (3) and R₂ in the general formula (4) are each an aliphatic chain having a main chain including an alkyleneoxy group or a polyalkyleneoxy group, and
the number of carbon atoms of an alkylene moiety of the alkyleneoxy group and the number of carbon atoms of an alkylene moiety of an alkyleneoxy unit constituting the polyalkyleneoxy group are 1 to 10 each.

5. The polyimide resin composition according to claim 1, wherein the (β2) aliphatic diamine represented by the general formula (3) is a compound represented by the following general formula (3-1), and the (β2) aliphatic diamine represented by the general formula (4) is a compound represented by the following general formula (4-1) 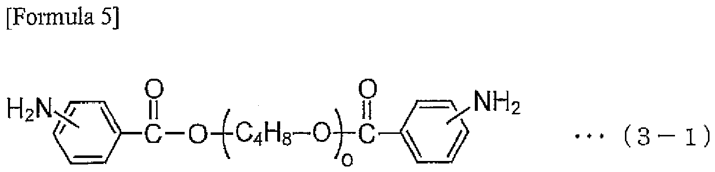 wherein in the formula (3-1), o represents an integer from 1 to 50, 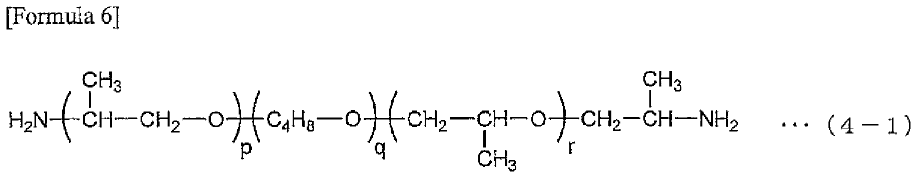 wherein, in the formula (4-1), p, q and r each independently represent an integer from 0 to 10, with the proviso that p+q+r is at least 1.

6. The polyimide resin composition according to claim 1, wherein the (β2) aliphatic diamine represented by the general formula (3) or (4) is present in an amount of 10 mol% to 45 mol% with respect to a total amount of the diamine constituting the polyimide.

7. The polyimide resin composition according to claim 1, wherein the (α1) aromatic tetracarboxylic acid dianhydride having a benzophenone backbone represented by the general formula (1) is at least one compound selected from the group consisting of 3,3',4,4'-benzophenone tetracarboxylic acid dianhydride, and 2,3,3',4-benzophenone tetracarboxylic acid dianhydride, and
the (β1) aromatic diamine having a benzophenone backbone represented by the general formula (2) is at least one compound selected from the group consisting of 3,3'-diaminobenzophenone, 3,4-diaminobenzophenone, and 4,4'-diaminobenzophenone.

8. The polyimide resin composition according claim 1, wherein the polyimide has a glass transition temperature of 120°C to less than 260°C.

9. The polyimide resin composition according claim 1, further comprising an inorganic filler.

10. The polyimide resin composition according claim 9, wherein the inorganic filler is a heat-dissipating filler, and
a volume of the heat-dissipating filler is 20 to 60vol% with respect to a total volume of the polyimide resin composition.

11. The polyimide resin composition according to claim 9, wherein the inorganic filler is an electroconductive filler and/or a magnetic filler, and
a total volume of the electroconductive filler and magnetic filler is 20 to 90vol% with respect to the total volume of the polyimide resin composition.

12. The polyimide resin composition according to claim 1, further comprising at least one compound selected from the group consisting of an epoxy compound, an acrylate compound, an isocyanate compound, a maleimide compound, and a nadimide compound.

13. A laminate comprising:
a substrate and;
a resin layer disposed on the substrate, the resin layer being formed of the polyimide resin composition according to any one of claims 1 to 12.

14. The laminate according to claim 13, wherein the substrate is made of metal, ceramic or resin.

15. An electronic circuit board comprising the laminate according to claim 13.

16. A semiconductor device comprising the laminate according to claim 13.

17. An electrode for a lithium-ion battery, comprising:
a metal foil; and
a layer disposed on the metal foil, the layer including an active substance and the polyimide resin composition according to any one of claims 1 to 12.

18. A separator for a lithium-ion battery comprising the polyimide resin composition according to any one of claims 1 to 12.

19. A heat-dissipating substrate comprising the polyimide resin composition according to claim 10.

20. An electromagnetic shielding substrate comprising the polyimide resin composition according to claim 11.

21. An adhesive for a surge part comprising the polyimide resin composition according to any one of claims 1 to 12.

22. A sealant for a surge part comprising the polyimide resin composition according to any one of claims 1 to 12.

23. An adhesive for a semiconductor manufacturing device comprising the polyimide resin composition according to any one of claims 1 to 12.

24. A dental material comprising the polyimide resin composition according to any one of claims 1 to 12.

25. A polyimide resin composition comprising a polyimide which is soluble in polar solvent, wherein
a polyimide film having a thickness of 50 µm which is formed of the polyimide resin composition satisfies the following conditions a) and b):
a) a storage modulus of elasticity E' measured at 180°C and a frequency of 1 Hz is 1,0 × 10⁵ Pa or more, and
b) an elongation rate at the time of tensile fracture at 23°C at a speed of 50 mm/min is 50% or more.
